# EUROPEAN PATENT SPECIFICATION

(11) **EP 3 810 108 B1**
(45) Date of publication and mention of the grant of the patent: **11.12.2024**
(21) Application number: 19742937.6
(22) Date of filing: 20.06.2019
(51) Int. Cl.: A61K 31/10, A61P 19/02, A61K 36/22, A61K 36/537

(54) **COMPOSITION FOR USE IN THE TREATMENT OF OSTEOARTHRITIS.**
ZUSAMMENSETZUNG ZUR VERWENDUNG BEI DER BEHANDLUNG VON ARTHROSE.
COMPOSITION POUR UTILISATION DANS LE TRAITEMENT DE L'ARTHROSE.

(30) Priority: 20.06.2018 IT 201800006474
(43) Date of publication of application: 28.04.2021
(73) Proprietor: Neilos S.r.l., 80063 Piano di Sorrento (NA) (IT)
(72) Inventor: DI MAIO, Umberto, 80065 Sant' Agnello ( Napoli) (IT)
(74) Representative: Di Giovine, Paolo
(86) International application number: PCT/IB2019/055194
(87) International publication number: WO 2019/244087

(56) References cited:
- WO-A2-2008/061720
- TANIDEH NADER ET AL: "The effect of hydroalcoholic extract ofMangifera indicaon induced osteoarthritis of knee in male guinea pigs", COMPARATIVE CLINICAL PATHOLOGY, SPRINGER-VERLAG, LONDON, vol. 25, no. 5, 11 July 2016 (2016-07-11), pages 973 - 979, XP036028237, ISSN: 1618-5641, [retrieved on 20160711], DOI: 10.1007/S00580-016-2289-Y
- SUKANYA SAHA ET AL: "Mangiferin: A xanthonoid with multipotent anti-inflammatory potential", BIOFACTORS, OXFORD UNIVERSITY PRESS, OXFORD, GB, vol. 42, no. 5, 24 May 2016 (2016-05-24), pages 459 - 474, XP071859805, ISSN: 0951-6433, DOI: 10.1002/BIOF.1292
- PATHAK N L ET AL: "Free radical scavenging activity of Mangifera indica methanolic extract in arthritic rats", INTERNATIONAL RESEARCH JOURNAL OF PHARMACY 2011 INTERNATIONAL RESEARCH JOURNAL OF PHARMACY IND, vol. 2, no. 6, June 2011 (2011-06-01), pages 148 - 152, XP009511640, ISSN: 2230-8407
- ABDEL-MOEIN N M ET AL: "Evaluation of the anti-inflammatory and anti-arthritic effects of some plant extracts", GRASAS Y ACEITES, vol. 62, no. 4, October 2011 (2011-10-01), pages 365 - 374, XP009511641
- JUNKO EZAKI ET AL: "Assessment of safety and efficacy of methylsulfonylmethane on bone and knee joints in osteoarthritis animal model", JOURNAL OF BONE AND MINERAL METABOLISM, SPRINGER-VERLAG, TO, vol. 31, no. 1, 10 August 2012 (2012-08-10), pages 16 - 25, XP035167155, ISSN: 1435-5604, DOI: 10.1007/S00774-012-0378-9
- MATTHEW BUTAWAN ET AL: "Methylsulfonylmethane: Applications and Safety of a Novel Dietary Supplement", NUTRIENTS, vol. 9, no. 3, 16 March 2017 (2017-03-16), pages 290, XP055564376, DOI: 10.3390/nu9030290

## Description

The present invention concerns a composition of substances, preferably obtained from natural sources, for use in the prevention and/or therapeutic treatment of osteoarthritis.

Osteoarthritis is a form of arthritis. The term "arthritis" refers to all disorders affecting the joints. In all cases the patients are affected by pain and stiffness of the joints. There are about 100 different types of arthritis of which the most common are rheumatoid arthritis and osteoarthritis.

Rheumatoid arthritis is an autoimmune disorder that mainly involves the joints, which become swollen and painful. The disease may also affect other parts of the body, leading for example to a reduction in the number of red blood cells, the appearance of inflammation around the heart and lungs, fever and a general sense of fatigue.

It is believed that this disease is due to a combination of genetic and environmental factors. The main mechanism involves the activation of the immune system against the joints, which leads to inflammation and thickening of the articular capsule, also involving the bone and cartilage. The initial site of the disease is the synovial membrane, where there is an infiltration of cells of the immune system. More specifically, rheumatoid arthritis is generally characterized by the succession of three phases: an initial phase due to non-specific inflammation, an amplification phase involving the activation of T cells, and finally a final phase of chronic inflammation due to the release of pro-inflammatory cytokines, such as interleukin-2, Υ-interferon, tumor necrosis factor (TNF) and interleukin-6.

The main risk factors for rheumatoid arthritis are gene mutations that involve disorders in the regulation of the immune response. One of the gene mutations responsible for the etiology of rheumatoid arthritis is localized in genes involved in the expression of the class 2 major histocompatibility complex (MHC) such as HLA DR4. Other documented environmental factors include, for example, smoking.

Osteoarthritis (osteoarthrosis or arthrosis) is a degenerative disease of the joints that affects millions of people around the world mainly in the hips, hands and knees. It is well known that the synovia, bone and cartilage are the tissues most involved in the pathological mechanisms of osteoarthritis.

The cause of the disease is likely to be related to changes in the homeostasis of the joint cartilage and bone that lead to the increase of destructive processes. In osteoarthritis there is a change in the structure of the bone and a degeneration of the joint cartilage. The subchondral bone becomes rigid, less capable of absorbing impact loads, which leads to greater stress on the cartilage. In fact, the main characteristics of the disease include a progressive loss of cartilage tissue, hypertrophic bone modifications and formation of osteophytes that grow at the edges of the bones involved.

With reference to cartilage, it is known that chondrocytes are the cells responsible for balancing the processes of synthesis and destruction of the matrix, capable of regulating cytokines and growth factors. In degenerative osteoarticular processes, this balance is usually compromised. In patients with osteoarthritis, chondrocytes produce high levels of inflammatory cytokines such as interleukin 1-β and tumor necrosis factor (TNF), which in turn reduce collagen synthesis and increase concentrations of catabolic mediators such as metalloproteases. At the same time, other pro-inflammatory substances such as interleukin-8 (IL-8) and interleukin-6 (IL-6), prostaglandin E2 and nitric oxide are released. The increase of oxidizing agents such as nitric oxide determines the apoptosis of chondrocytes and thus the degeneration of the matrix.

In addition to the inflammatory process, another event typically found in osteoarthritis is a progressive reduction in the level of proteoglycans. Without the protective effect of proteoglycans, the cartilage becomes much more susceptible to degeneration and degradation. Aggrecan is considered the main proteoglycan responsible for providing hydration and elasticity to cartilage tissues, and, in patients with osteoarthritis, a reduction in its concentration is observed.

Several studies have moreover shown that chondrocytes are also responsible for the production of reactive oxygen species (ROS), such as superoxide anions (O²⁻) and hydroxyl radicals (OH⁻). This effect is mainly due to the activation of macrophages and neutrophils, which take part in the inflammatory response.

The most documented risk factors for osteoarthritis are age, sex, obesity, genetics and trauma which may determine the onset of the pathophysiology of osteoarthritis. Age is the main risk factor, as it is known that with increasing age the tensile properties of the articular cartilage decrease, leading to mechanical problems. Women normally show greater pain and disability than men. People with a high body mass index are more at risk.

The most common symptoms of osteoarthritis include chronic pain, due to an increased concentration at the affected sites of excitatory amino acids such as glutamate, which contributes to hyperalgesia and pain. Another characteristic symptom is articular rigidity due to a decrease in the levels of surfactant phospholipids responsible for reducing friction by ensuring optimal lubrication.

Due to changes in, for example, the articular cartilage in the knee, additional effects appear in patients, including edema of the underlying soft tissues, blood circulation disorders, bone enlargement and swelling.

The pharmacological treatments traditionally used in osteoarticular diseases are primarily represented by analgesic drugs, which help to reduce the sensation of pain but, obviously, do not represent anything but symptomatic treatments. Anti-inflammatory drugs of various kinds (non-steroidal and steroidal) are also used to counteract the inflammatory processes that characterize the disease. However, these drugs require numerous daily administrations to reach adequate therapeutic doses and often have serious side effects, such as peptic ulcers or gastric perforations. The use of selective COX-2 NSAIDs (such as Celecoxib) reduces this type of event, but at the same time may cause serious damage to the cardiovascular system. Paracetamol has fewer side effects but is only indicated in mild to moderate forms of osteoarthritis.

JUNKO EZAKI ET AL: "Assessment of safety and efficacy of methylsulfonylmethane on bone and knee joints in osteoarthritis animal model)", JOURNAL OF BONE AND MINERAL METABOLISM, SPRINGER-VERLAG, TO, vol. 31, no. 1, 10 August 2012 (2012-08-10), pages 16-25 mentions that methylsulfonylmethane (MSM) intake improved total arthritic score and decreased degeneration of cartilage in the OA model mice in a dose-dependent manner.

WO 2008/061720 A2 relates to compounds that can be used in the treatment of inflammatory disorder and or joint disorders such as OA, and mentions a number of substances which can be obtained from plants like sage and other *Salvia* sp.

TANIDEH NADER ET AL: "The effect of hydroalcoholic extract of Mangifera indica on induced osteoarthritis of knee in male guinea pigs", COMPARATIVE CLINICAL PATHOLOGY, SPRINGER-VERLAG, LONDON, vol. 25, no. 5, 11 July 2016 (2016-07-11), pages 973-979 relates to mangiferin and mentions its applicability in the treatment of osteoarthritis.

There is therefore a need to provide treatments, alternative to those already existing, that are effective in the prevention and/or therapeutic treatment of osteoarthritis but that do not have the side effects and/or disadvantages of treatments in the state of the art.

These and other needs are satisfied by the present invention, which provides a composition characterized in that it comprises a synergistic combination of active ingredients, obtained from natural sources, the aforesaid combination having proved particularly effective against osteoarthritis.

The composition of the invention is as defined in the appended claim 1. Further features and advantages of the invention are defined in the dependent claims. The claims form an integral part of the present description.

Hereinafter, a detailed description of some of the preferred embodiments of the invention is provided.

The synergistic composition of the present invention is used for the treatment and prevention of osteoarthritis.

In the composition of the present invention, the synergistic action takes place between methylsulfonylmethane (MSM), at least one extract of a plant belonging to the genus *Salvia,* preferably at least one extract of *Salvia officinalis,* and one extract of *Mangifera indica* (mango).

The scientific name of common sage is *Salvia officinalis,* an aromatic plant belonging to the Lamiaceae family, native to the Mediterranean regions. It is an evergreen shrub with woody stems and greyish leaves with blue-purple flowers. Its name comes from the Latin *salus salvus,* which means healthy, in good health, precisely in reference to its healing properties. In traditional medicine, its use for its carminative, expectorant, antidiabetic and antidepressant properties is particularly well known.

Within the scope of the present invention, of particular interest is its anti-inflammatory and antioxidant activity, therapeutic effects that are desirable in the treatment of osteoarticular diseases. These effects are attributed to the flavonoids and phenolic compounds present in the extracts of *Salvia officinalis* and other plants of the genus *Salvia.* Studies in the literature show that compounds with terpenic structure (e.g. carnosol, carnosic acid, carnosic acid 12-methyl ether, ursolic acid) of *Salvia officinalis* have a specific anti-inflammatory effect in cultures of human macrophages and chondrocytes stimulated with LPS. In these studies, *Salvia officinalis* extracts show anti-inflammatory effects, reducing the production of pro-inflammatory cytokines by acting on the genes involved in their transcription, and, in particular, they act at the level of cyclooxygenases and mPGES-1.

*Sage* compounds, and in particular carnosol, rosmarinic acid and carnosic acid, have a high antioxidant power and are able to significantly increase the action of superoxide dismutase (SOD), catalase and glutathione peroxidase in rats.

Due to their multiple properties (anti-inflammatory, antinociceptive and antioxidant), *Salvia officinalis* extracts are thus of considerable interest for the development of nutraceutical products useful in the prevention and treatment of osteoarticular diseases characterized by inflammation, pain and oxidative stress.

*Mangifera indica,* also known as mango, is a plant belonging to the Anacardiaceae family cultivated throughout the tropical and subtropical world for the sale of the fruit. It is an evergreen tree that grows in height between 10 and 45 meters. The most familiar parts are the fruits, drupes which can vary in shape, size and color. Several components of the plant have always been used in traditional medicine as astringents, stomachics (digestive stimulators), tonics, laxatives, diuretics, and for the treatment of diarrhea, anemia, asthma.

The main constituent classes of the plant are polyphenols, flavonoids, triterpenoids and the main compounds are mangiferin, isomangiferin, and tannin derivatives and gallic acid. Mangiferin, a xanthone C-glucoside, is the compound that shows the greatest antioxidant, anti-inflammatory, immunomodulating, antidiabetic, antimicrobial, antiviral, hepatoprotective, anti-allergic and anti-tumor properties.

In the scope of the present invention, the anti-inflammatory and antioxidant action of mangiferin and mango extract is of particular importance. Anti-inflammatory activity has been confirmed by *in vivo* studies on mice in carrageenan edema models and neuropathic pain.

In humans, *Mangifera indica* extract is able to significantly inhibit levels of phospholipase A2 (PLA2) in synovial fluid thus blocking the initiation of the arachidonic acid cascade and the production of pro-inflammatory mediators, resulting in a potentially useful analgesic action in patients with arthritis. An important study described in the literature shows the specific anti-inflammatory effect of mangiferin in human chondrocyte cultures. In this study, mangiferin is able to prevent the increase of inflammatory mediators induced by cell stimulation with IL-1β (PGE2, nitric oxide "NO", metalloproteinase "MMP" and activation of NF-xB). This action would be mediated by the ability of mangiferin to directly activate the nuclear receptor PPARγ, which in turn would result in an inhibition of inflammation.

In a recent study, the components of mango were found to inhibit the expression of interleukin 17 and increase the expression of interleukin 10 with anti-inflammatory action in *in vivo* inflammation models.

A clinical trial conducted in 2014 in patients with osteoarthritis showed the beneficial effect of supplementation with *Mangifera indica* extract in combination with methotrexate on rheumatoid arthritis symptoms (900 mg daily for 180 days in 10 patients).

Methylsulfonylmethane (MSM) is an organic compound containing sulfur. Studies on the anti-inflammatory and antioxidant action of MSM show that, *in vitro,* this compound is able to directly inhibit the pathway of the transcription factor NF-κB, reducing the levels of pro-inflammatory cytokines IL-1 and IL-6. MSM also inhibits TNF-α and the enzyme COX2. This would explain its anti-inflammatory and antioxidant action. In fact, the reduced expression of the aforesaid factors determines a decrease in enzymes and cytokines involved in the production of free radicals (ROS). In addition, the production of superoxide anions (O²⁻), NO and TNF-α is significantly reduced. This results in an inhibition of ROS production by the mitochondria. In various studies in the literature, the efficacy of MSM in osteoarticular diseases has been demonstrated, particularly in arthritis of the knee and in pain caused by arthritis in general. In such studies, the compound is used alone or in combination with other substances and is free of side effects. In particular, it has been shown that taking MSM orally (1.125g - 3 times daily for 12 weeks in 140 patients) results in a marked improvement in physical function and a significant reduction in pain in patients with osteoarthritis. Interesting data also emerge from a clinical trial where it is shown that MSM alone (3g twice daily for 12 weeks in 50 patients) significantly improves pain symptoms in patients with osteoarthritis. In another clinical study, the efficacy of MSM in combination with boswellic acid (BA) (5g of MSM + 7.2mg of BA for 6 months in 120 patients) in the treatment of osteoarthritis is demonstrated.

The important anti-inflammatory properties of MSM make it a particularly interesting component for the development of alternative products to traditional pharmacological treatments for osteoarticular diseases.

As indicated, the synergistic composition of the present invention is effective in the treatment and/or prevention of osteoarticular diseases. To summarize, the effectiveness of the composition derives from the following activities of the components:
- extracts of plants belonging to the genus *Salvia* and in particular *Salvia officinalis* (carnosol, carnosic acid, carnosic acid 12-methyl ether, ursolic acid) have a specific anti-inflammatory effect on chondrocytes, primarily involved in the onset and progression of arthritis, by acting both at the level of cyclooxygenase and at the level of mPGES-1;
- the extract of the mango plant, in particular mangiferin, has an important antioxidant, anti-inflammatory and antinociceptive activity due to its inhibiting action on PLA 2 in synovial fluid and its inhibiting action on interleukin 17;
- MSM blocks the NF-xB pathway upstream, reducing levels of pro-inflammatory cytokines (IL-1 and IL-6) and specifically inhibits the production of mitochondrial ROS.

As stated previously, in the present invention, the synergistic action takes place between methylsulfonylmethane (MSM), at least one extract of a plant belonging to the genus *Salvia,* preferably at least one extract of *Salvia officinalis,* and an extract of *Mangifera indica* (mango).

In a preferred embodiment, MSM is administered in a daily dose of between 50 mg and 10 g, for example by administering a dosage form wherein MSM is present in a quantity of between 5 and 95% w/w, more preferably in a quantity of between 15 and 85% by weight on the total weight of the composition; at least one extract of a plant belonging to the genus *Salvia,* preferably at least one extract of *Salvia officinalis,* is administered in a daily dose of between 1 mg and 5000 mg, for example by administration of a dosage form wherein the aforesaid at least one extract is present in a quantity of between 0.5 and 30% w/w, more preferably in a quantity of between 1 and 15% by weight on the total weight of the composition; *Mangifera indica* (mango) extract is administered in a daily dose of between 1 mg and 5000 mg, for example by administration of a dosage form wherein the aforesaid extract is present in a quantity of between 1 and 50% w/w, more preferably in a quantity of between 3 and 20% by weight on the total weight of the composition.

The dosage form may be a pharmaceutical composition or a food supplement or a medical device or a food for special medical purposes including the aforementioned active ingredients in a mixture. The preferred route of administration is oral or topical.

The following examples are provided purely for illustrative purposes and are not intended to limit the scope of the invention as defined in the appended claims.

### EXAMPLES

The following examples concern any form of pharmaceutical dosage that is acceptable, preferably oral or topical, suitable in the scope of the present invention.

Some examples of formulations are given, with the quantities of the relative active substances present in each dosage unit.

### EXAMPLE 1

| Active ingredient | Daily dose |
|---|---|
| Methylsulfonylmethane | 4 g |
| *Salvia officinalis* e.s. | 300 mg |
| *Mangifera indica* e.s. | 500 mg |

### EXAMPLE 2

| Active ingredient | Daily dose |
|---|---|
| Methylsulfonylmethane | 6 g |
| *Salvia officinalis* e.s. | 150 mg |
| *Mangifera indica* e.s. | 250 mg |

### EXAMPLE 3

| Active ingredient | Daily dose |
|---|---|
| Methylsulfonylmethane | 3 g |
| *Salvia officinalis* e.s. | 200 mg |
| *Mangifera indica* e.s. | 350 mg |

### EXAMPLE 4

| Active ingredient | Daily dose |
|---|---|
| Methylsulfonylmethane | 500 mg |
| *Salvia officinalis* e.s. | 100 mg |
| *Mangifera indica* e.s. | 100 mg |

### EXPERIMENTAL PART

The synergistic action of the composition with respect to the individual active ingredients is evaluated using experimental methods *in vitro* and/or *in vivo.*

Various cell studies are able to provide important information about the anti-inflammatory capacity of bioactive compounds able to inhibit cytokine expression. IC50 studies are able to provide information for cellular inhibition. To evaluate in more detail the response (inhibition/activation) of cytokines (TNF-α, IL-1, IL-4, IL-6, IL-8, IL-10,IL-17, INF-Y, COMP) during inflammatory processes induced by LPS, the "Whole Blood Assay (WBA)" or the "peripheral blood mononuclear cell" (PBMC) test or other similar tests are used.

As an *in vivo* test, a simple model of general inflammation is the model of carrageenan-induced edema in mice or rats. In this test, the injection of carrageenan into the animal's paw results in an acute inflammatory reaction of the paw with the appearance of classic signs of inflammation; this reaction reaches its maximum within 3 hours of inoculation. This test may be used to assess the ability of the active ingredients of the present invention to reduce the edema of the paw induced by the injection of carrageenan.

More complex methods that are used for the evaluation of osteoarticular diseases are for example the model of arthritis induced by collagen and arthritis induced by Freund's adjuvant, which allow evaluating the effects of potential agents in the treatment of arthritis over a prolonged period of time.

## Claims

1. A composition comprising methylsulfonylmethane (MSM), at least one extract of a plant belonging to the genus *Salvia,* and an extract of *Mangifera indica* (mango), for use in the prevention and/or therapeutic treatment of osteoarthritis.

2. The composition for use according to claim 1, wherein the methylsulfonylmethane (MSM) is present in the composition in an amount ranging from 5 to 95% by weight on the total weight of the composition.

3. The composition for use according to claim 2, wherein the methylsulfonylmethane (MSM) is present in the composition in an amount ranging from 15 to 85% by weight on the total weight of the composition.

4. The composition for use according to any of claims 1 to 3, wherein the at least one extract of a plant belonging to the genus *Salvia* is present in the composition in an amount ranging from 0.5 to 30% by weight on the total weight of the composition.

5. The composition for use according to claim 4, wherein the at least one extract of a plant belonging to the genus *Salvia* is present in the composition in an amount ranging from 1 to 15% by weight on the total weight of the composition.

6. The composition for use according to any one of claims 1 to 5, wherein the *Mangifera indica* (mango) extract is present in the composition in an amount ranging from 1 to 50% by weight on the total weight of the composition.

7. The composition for use according to claim 6, wherein the *Mangifera indica* (mango) extract is present in the composition in an amount ranging from 3 to 20% by weight on the total weight of the composition.

8. The composition for use according to any one of claims 1 to 7, wherein the plant belonging to the genus *Salvia* is *Salvia officinalis.*

9. The composition for use according to any one of claims 1 to 8, which is a pharmaceutical composition, a food supplement, a medical device, a food for special medical purposes, a cosmetic, for oral or topical administration.

## Patentansprüche

1. Zusammensetzung, umfassend Methylsulfonylmethan (MSM), mindestens einen Extrakt einer Pflanze, die zu der Gattung *Salbei* gehört, und einen Extrakt aus *Mangifera indica* (Mango), zur Verwendung bei der Vorbeugung und/oder therapeutischen Behandlung von Osteoarthritis.

2. Zusammensetzung zur Verwendung nach Anspruch 1, wobei das Methylsulfonylmethan (MSM) in der Zusammensetzung in einer Menge in einem Bereich von 5 bis 95 Gew.-% des Gesamtgewichts der Zusammensetzung vorhanden ist.

3. Zusammensetzung zur Verwendung nach Anspruch 2, wobei das Methylsulfonylmethan (MSM) in der Zusammensetzung in einer Menge in einem Bereich von 15 bis 85 Gew.-% des Gesamtgewichts der Zusammensetzung vorhanden ist.

4. Zusammensetzung zur Verwendung nach einem der Ansprüche 1 bis 3, wobei der mindestens eine Extrakt einer Pflanze, die zu der Gattung *Salbei* gehört, in der Zusammensetzung in einer Menge in einem Bereich von 0,5 bis 30 Gew.-% des Gesamtgewichts der Zusammensetzung vorhanden ist.

5. Zusammensetzung zur Verwendung nach Anspruch 4, wobei der mindestens eine Extrakt einer Pflanze, die zu der Gattung *Salbei* gehört, in der Zusammensetzung in einer Menge in einem Bereich von 1 bis 15 Gew.-% des Gesamtgewichts der Zusammensetzung vorhanden ist.

6. Zusammensetzung zur Verwendung nach einem der Ansprüche 1 bis 5, wobei der Extrakt aus *Mangifera indica* (Mango) in der Zusammensetzung in einer Menge in einem Bereich von 1 bis 50 Gew.-% des Gesamtgewichts der Zusammensetzung vorhanden ist.

7. Zusammensetzung zur Verwendung nach Anspruch 6, wobei der Extrakt aus *Mangifera indica* (Mango) in der Zusammensetzung in einer Menge in einem Bereich von 3 bis 20 Gew.-% des Gesamtgewichts der Zusammensetzung vorhanden ist.

8. Zusammensetzung zur Verwendung nach einem der Ansprüche 1 bis 7, wobei die Pflanze, die zu der Gattung *Salbei* gehört, *Salvia officinalis* ist.

9. Zusammensetzung zur Verwendung nach einem der Ansprüche 1 bis 8, die eine pharmazeutische Zusammensetzung, ein Nahrungsergänzungsmittel, eine Medizinvorrichtung, ein Lebensmittel für besondere medizinische Zwecke, ein Kosmetikum, für eine orale oder eine topische Verabreichung ist.

## Revendications

1. Composition comprenant du méthylsulfonylméthane (MSM), au moins un extrait d'une plante appartenant au genre *Salvia,* et un extrait de *Mangifera indica* (mangue), pour utilisation dans la prévention et/ou le traitement thérapeutique de l'arthrose.

2. Composition pour utilisation selon la revendication 1, dans laquelle le méthylsulfonylméthane (MSM) est présent dans la composition en une quantité allant de 5 à 95 % en poids par rapport au poids total de la composition.

3. Composition pour utilisation selon la revendication 2, dans laquelle le méthylsulfonylméthane (MSM) est présent dans la composition en une quantité allant de 15 à 85 % en poids par rapport au poids total de la composition.

4. Composition pour utilisation selon l'une quelconque des revendications 1 à 3, dans laquelle l'au moins un extrait d'une plante appartenant au genre *Salvia* est présent dans la composition en une quantité allant de 0,5 à 30 % en poids par rapport au poids total de la composition.

5. Composition pour utilisation selon la revendication 4, dans laquelle l'au moins un extrait d'une plante appartenant au genre *Salvia* est présent dans la composition en une quantité allant de 1 à 15 % en poids par rapport au poids total de la composition.

6. Composition pour utilisation selon l'une quelconque des revendications 1 à 5, dans laquelle l'extrait de *Mangifera indica* (mangue) est présent dans la composition en une quantité allant de 1 à 50 % en poids par rapport au poids total de la composition.

7. Composition pour utilisation selon la revendication 6, dans laquelle l'extrait de *Mangifera indica* (mangue) est présent dans la composition en une quantité allant de 3 à 20 % en poids par rapport au poids total de la composition.

8. Composition selon l'une quelconque des quelconques revendications 1 à 7, dans laquelle la plante
appartenant au genre *Salvia* est *Salvia officinalis.*

9. Composition pour utilisation selon l'une quelconque des revendications 1 à 8, qui est une composition pharmaceutique, un complément alimentaire, un dispositif médical, un aliment destiné à des fins médicales spéciales, un cosmétique, pour une administration orale ou topique.
